# EUROPEAN PATENT APPLICATION

(11) **EP 3 974 009 A1**
(43) Date of publication of application: **30.03.2022**
(21) Application number: 21191269.6
(22) Date of filing: 13.08.2021
(51) Int. Cl.: A61M 1/36, B04B 5/04

(54) **CENTRIFUGE BOWL AND BLOOD CENTRIFUGE SYSTEM**

(30) Priority: 26.09.2020 US 202063083866 P; 01.02.2021 TW 110103693
(71) Applicant: Sangtech Lab Inc., Taipei (TW)
(72) Inventor: LIANG, Cheng-Sheng, Taipei (TW); LIANG, Tao, Taipei (TW); KE, Hsin Tai, Taipei (TW)
(74) Representative: Becker, Eberhard

(57) **Abstract**

A centrifuge bowl (100) separates a first component and a second component in a sample and includes a shell (110), a core (120), a separation cavity (130), and a stator head (140). The shell includes an upper shell part (111), a middle shell part (112), a lower shell part (113), and a bottom shell part (114). The separation cavity is arranged between the lower shell part and the core. The stator head is arranged on the shell and includes an input tube (141) and an output tube (142). The sample enters the separation cavity via the input tube. When the shell and the core rotate on a rotation axis (R), the sample in the separation cavity is separated into the first component and the second component according to a magnitude of an inertial force. In addition, a blood centrifuge system including the above centrifuge bowl is provided.

## Description

### BACKGROUND

### [Technical Field]

The disclosure relates to a centrifuge bowl and a blood centrifuge system, and particularly relates to a centrifuge bowl and a blood centrifuge system which can separate blood automatically or achieve better separation effect.

### [Description of Related Art]

Generally, the method for separating plasma from blood substantially includes the following steps. A blood sample of 8 to 10 ml is taken and added into a test tube in the laboratory. Next, centrifugal separation is performed in an experimental centrifuge to separate plasma, platelets, and red blood cells into upper and lower layers in the test tube. After centrifugation, the upper layer of plasma and platelets is collected manually by the medical staff. Specifically, since the collection process is fully manual, it is impossible to control the concentration and the collected amount of platelets collected, and it is impossible to prevent the blood from being contaminated by external sources.

### SUMMARY

The disclosure provides a centrifuge bowl and a blood centrifuge system which can separate blood automatically or achieve better separation effect.

A centrifuge bowl according to the disclosure is configured to separate a first component and a second component in a sample. The centrifuge bowl includes a shell, a core, a separation cavity, and a stator head. The shell includes an upper shell part, a middle shell part, a lower shell part, and a bottom shell part. The middle shell part connects the upper shell part and the lower shell part, and the lower shell part connects the middle shell part and the bottom shell part. The core is arranged in the shell. The separation cavity is arranged between the lower shell part and the core. The stator head is arranged on the shell and includes an input tube and an output tube. The sample enters the separation cavity via the input tube. When the shell and the core rotate on a rotation axis, the sample in the separation cavity is separated into the first component and the second component according to a magnitude of an inertial force.

In an embodiment of the disclosure, the separation cavity is defined as being surrounded by the core, the middle shell part, the lower shell part, and the bottom shell part.

In an embodiment of the disclosure, when a first inertial force of the first component is greater than a second inertial force of the second component, the separated first component is away from the core, and the separated second component is adjacent to the core.

In an embodiment of the disclosure, a first angle is present between an outer side-surface of the core and a first direction parallel to the rotation axis, a second angle is present between an outer surface of the lower shell part and the first direction, and the second angle is greater than the first angle.

In an embodiment of the disclosure, the first angle is less than 90 degrees, and the second angle is less than 90 degrees.

In an embodiment of the disclosure, a third angle is present between an inner surface of the bottom shell part and the first direction, and the third angle is greater than 90 degrees.

In an embodiment of the disclosure, a fourth angle is present between an outer surface of the middle shell part and the first direction, the fourth angle is less than 90 degrees, and the fourth angle is greater than the second angle.

In an embodiment of the disclosure, the separation cavity has a recessed region, and the recessed region is defined by a contour formed upon connection of the lower shell part and the bottom shell part.

In an embodiment of the disclosure, the sample is blood, the first component includes red blood cells, and the second component includes platelets and plasma.

A blood centrifuge system according to the disclosure includes the above centrifuge bowl.

Based on the above, in the centrifuge bowl and the blood centrifuge system according to an embodiment of the disclosure, due to the difference in the magnitude of the initial force of the first component and the second component in the sample, when the shell and the core rotate on the rotation axis, the sample can be automatically separated into the first component and the second component in the separation cavity according to the magnitude of the inertial force. Accordingly, the centrifuge bowl and the blood centrifuge system according to an embodiment of the disclosure can achieve the effect of automatically separating blood.

To make the aforementioned more comprehensible, several embodiments accompanied with drawings are described in detail as follows.

### BRIEF DESCRIPTION OF THE DRAWINGS

FIG. 1 is a schematic cross-sectional view of a centrifuge bowl according to an embodiment of the disclosure.
FIG. 2A to FIG. 2D are schematic cross-sectional views showing separation of a sample using the centrifuge bowl of FIG. 1.
FIG. 3 is a schematic cross-sectional view of a blood centrifuge system according to an embodiment of the disclosure.

### DESCRIPTION OF THE EMBODIMENTS

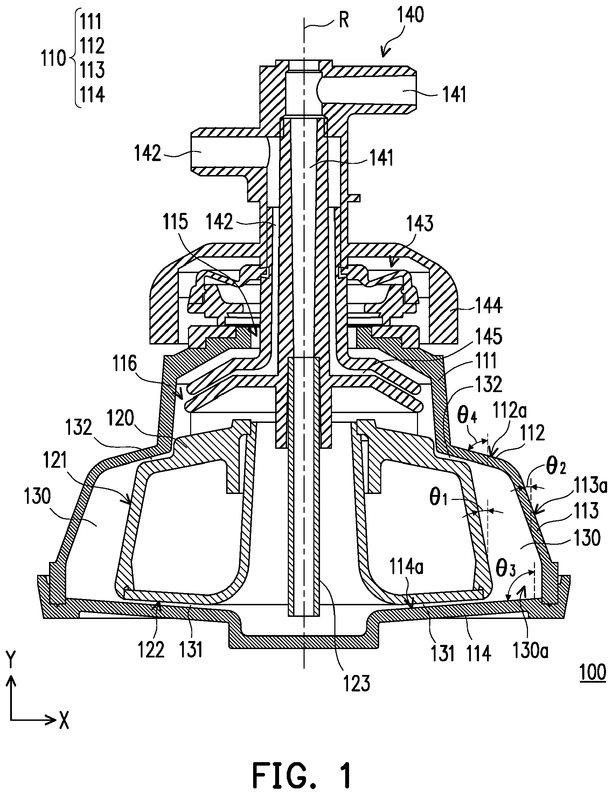

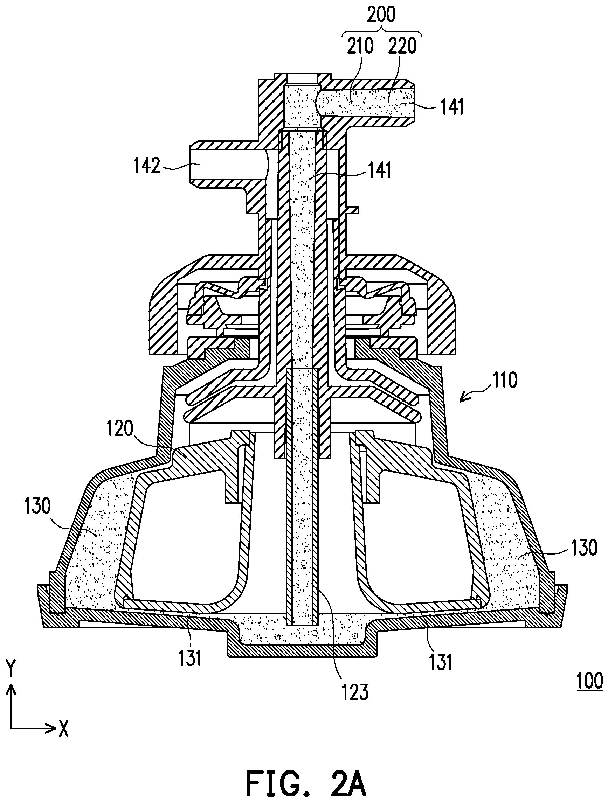

FIG. 1 is a schematic cross-sectional view of a centrifuge bowl according to an embodiment of the disclosure. FIG. 2A to FIG. 2D are schematic cross-sectional views showing separation of a sample using the centrifuge bowl of FIG. 1. Referring to FIG. 1 first, a centrifuge bowl 100 according to this embodiment includes a shell 110, a core 120, a separation cavity 130, and a stator head 140. The core 120 is arranged in the shell 110, the separation cavity 130 is arranged between the shell 110 and the core 120, and the stator head 140 is arranged on the shell 110.

Specifically, in this embodiment, the shell 110 includes an upper shell part 111, a middle shell part 112, a lower shell part 113, a bottom shell part 114, an opening 115, and an internal space 116. The middle shell part 112 connects the upper shell part 111 and the lower shell part 113, and the lower shell part 113 connects the middle shell part 112 and the bottom shell part 114. The middle shell part 112 may extend outward from its junction with the upper shell part 111, for example, in a direction substantially away from a rotation axis R, to further connect to the lower shell part 113. The rotation axis R is an imaginary rotation center located at the center of the shell 110, so that the shell 110 and the core 120 can rotate together on the rotation axis R during centrifugation of the centrifuge bowl 100. Therefore, the shell 110 and the core 120 may be regarded as movers in the centrifuge bowl 100.

In addition, in this embodiment, the shape of the shell 110 may be, for example, a bell shape, but is not limited thereto. The opening 115 is arranged at the top of the shell 110 and is surrounded by the upper shell part 111. The internal space 116 is surrounded by the upper shell part 111, the middle shell part 112, the lower shell part 113, and the bottom shell part 114 to form a hollow part. The opening 115 may communicate with the internal space 116.

In this embodiment, the core 120 is arranged in the internal space 116 of the shell 110. The core 120 has an outer side-surface 121, an outer bottom-surface 122, and a central sleeve 123. The central sleeve 123 penetrates the core 120. One end of the central sleeve 123 away from the opening 115 passes through the outer bottom-surface 122 of the core 120 so that the end can be close to the bottom shell part 114 of the shell 110. The central sleeve 123 does not contact the bottom shell part 114. In this embodiment, an included angle between the outer side-surface 121 of the core 120 and a first direction Y parallel to the rotation axis R is a first angle θ₁. The first angle θ₁ is, for example, greater than 0 degrees and less than 90 degrees, but is not limited thereto.

In addition, in this embodiment, multiple cavities are present between the core 120 and the shell 110, and these cavities at least include the separation cavity 130, a first flow channel 131, and a second flow channel 132. The separation cavity 130 is arranged between the lower shell part 113 of the shell 110 and the core 120. In some embodiments, the separation cavity 130 may be defined as being surrounded by the core 120, the middle shell part 112, the lower shell part 113, and the bottom shell part 114. In this embodiment, the separation cavity 130 has a recessed region 130a. The recessed region 130a is away from the core 120 and is adjacent to the lower shell part 113. The recessed region 130a may be defined by a contour formed upon connection of the lower shell part 113 and the bottom shell part 114. In this embodiment, the first flow channel 131 is arranged between the outer bottom-surface 122 of the core 120 and an inner surface 114a of the bottom shell part 114. The second flow channel 132 is arranged between the core 120 and the middle shell part 112 of the shell 110. The first flow channel 131 communicates with the separation cavity 130, and the second flow channel 132 also communicates with the separation cavity 130.

Moreover, in this embodiment, an included angle between an outer surface 113a of the lower shell part 113 and the first direction Y is a second angle θ₂. The second angle θ₂ is, for example, greater than 0 degrees and less than 90 degrees, but is not limited thereto. The second angle θ₂ is, for example, greater than the first angle θ₁, but is not limited thereto. In this embodiment, an included angle between the inner surface 114a of the bottom shell part 114 and the first direction Y is a third angle θ₃. The third angle θ₃ is, for example, greater than 90 degrees and less than 180 degrees, but is not limited thereto. In this embodiment, an included angle between an outer surface 112a of the middle shell part 112 and the first direction Y is a fourth angle θ₄. The fourth angle θ₄ is, for example, greater than 0 degrees and less than 90 degrees, but is not limited thereto. The fourth angle θ₄ is, for example, greater than the second angle θ₂.

In this embodiment, the stator head 140 is arranged on the upper shell part 111 of the shell 110. During centrifugation of the centrifuge bowl 100, the stator head 140 is stationary and does not rotate on the rotation axis R. The stator head 140 includes an input tube 141, an output tube 142, a sealing gasket 143, a shell cover 144, and a third flow channel 145. Specifically, the input tube 141 may pass through the opening 115 of the shell 110 to extend and connect to the central sleeve 123 of the core 120. The output tube 142 is wrapped around the input tube 141 and passes through the opening 115 of the shell 110 to be connected to the third flow channel 145. The third flow channel 145 may be connected to the second flow channel 132. The sealing gasket 143 is arranged on the upper shell part 111 to contact the upper shell part 111 and cover a part of the opening 115 exposed by the output tube 142. The shell cover 144 is wrapped around the sealing gasket 143.

The structural design of the centrifuge bowl 100 of this embodiment has been described above. Next, referring to FIG. 1 and FIG. 2A to FIG. 2D at the same time, the following description will illustrate how the centrifuge bowl 100 of this embodiment is used to automatically separate a first component 210 and a second component 220 in a sample 200.

First, referring to FIG. 2A, the sample 200 is fed in via the input tube 141, so that the sample 200 can pass through the central sleeve 123 and the first flow channel 131 and enter the separation cavity 130. In this embodiment, the sample 200 is, for example, blood or other liquid samples, but is not limited thereto. In addition, when the sample 200 is blood, the first component 210 in the sample 200 may include, for example, red blood cells, and the second component 220 may include, for example, platelets and plasma, but the disclosure is not limited thereto.

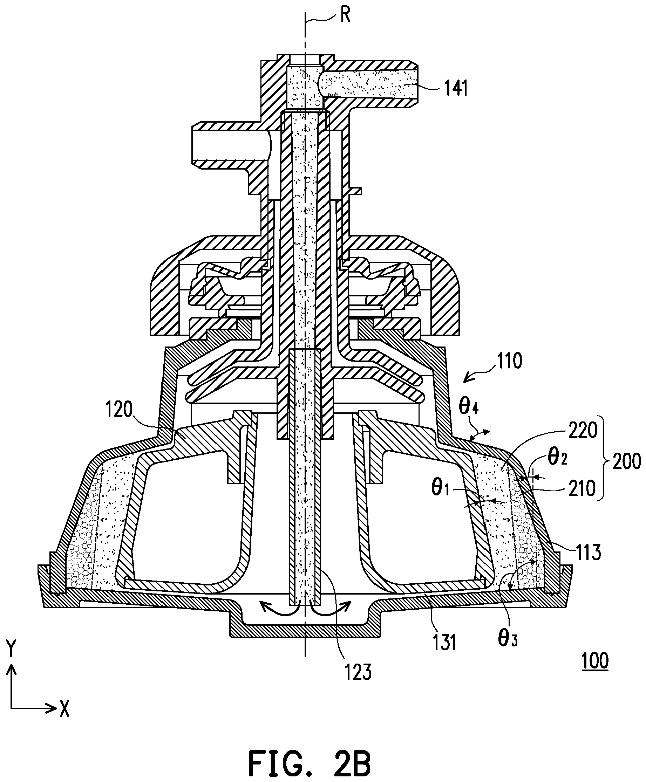

Next, referring to FIG. 2B, centrifugation of the centrifuge bowl 100 is performed, so that the shell 110 and the core 120 may rotate on the rotation axis R. At this time, when the shell 110 and the core 120 rotate on the rotation axis R, the sample 200 in the separation cavity 130 may be separated into the first component 210 and the second component 220 according to the magnitude of the inertial force. When a first inertial force of the first component 210 is greater than a second inertial force of the second component 220, the separated first component 210 may be moved away from the core 120, and the separated second component 220 may be adjacent to the core 120.

Specifically, during centrifugation of the centrifuge bowl 100, different inertial forces may be generated for the first component 210 and the second component 220 in the sample 200 due to the centrifugal force, so that the first component 210 and the second component 220 may settle and separate according to the direction of the inertial force. Specifically, the direction of the centrifugal force and the direction of the inertial force are substantially perpendicular to the rotation axis R (or the first direction Y), and for the separation cavity 130 on the right side in FIG. 2B, the direction of the centrifugal force and the direction of the inertial force are, for example, a second direction X perpendicular to the first direction Y. Then, since the density of the first component 210 is greater than the density of the second component 220, the first inertial force of the first component 210 may be greater than the second inertial force of the second component 220, and a first sedimentation velocity of the first component 210 may be greater than a second sedimentation velocity of the second component 220. Further, since the first inertial force of the first component 210 may be greater than the second inertial force of the second component 220 (or the first sedimentation velocity of the first component 210 may be greater than the second sedimentation velocity of the second component 220), the first component 210 and the second component 220 in the sample 200 may be automatically separated into two layers in the separation cavity 130, so that the separated first component 210 (first layer) may be away from the core 120 (or may be adjacent to the lower shell part 113), and the separated second component 220 (second layer) may be adjacent to the core 120 (or may be away from the lower shell part 113). In some embodiments, the separated first component 210 may be substantially concentrated in the recessed region 130a of the separation cavity 130, but is not limited thereto.

In addition, during centrifugation of the centrifuge bowl 100, since the third angle θ₃ may be greater than 90 degrees, the sample 200 flowing from the first flow channel 131 to the separation cavity 130 may also have a first flow velocity of flowing substantially toward the first direction Y. As shown in FIG. 2B, since the first sedimentation velocity (or the first inertial force) of the first component 210 adjacent to the lower shell part 113 is greater than the first flow velocity, the separated first component 210 settles in the direction of the first sedimentation velocity (i.e., the direction of the first inertial force) to concentrate in the recessed region 130a and does not easily enter the second flow channel 132. Conversely, since the second sedimentation velocity (or the second inertial force) of the second component 220 adjacent to the core 120 is less than the first flow velocity, the separated second component 220 flows in the direction of a second flow velocity and enters the second flow channel 132.

It is noted that in this embodiment, since the first component 210 is adjacent to the lower shell part 113 and the second angle θ₂ may be greater than the first angle θ₁, the second flow velocity of the first component 210 itself is further less than the first flow velocity of the flow of the sample 200, and the first sedimentation velocity (or the first inertial force) is further greater than the second flow velocity. Accordingly, the separated first component 210 can more easily settle in the direction of the first sedimentation velocity (i.e., the direction of the first inertial force) and concentrate in the recessed region 130a, and can be less likely to enter the second flow channel 132. In other words, since the second angle θ₂ may be greater than the first angle θ₁, the second flow velocity at which the first component 210 adjacent to the lower shell part 113 flows toward the second flow channel 132 is further reduced, and thereby the first component 210 can more easily concentrate in the recessed region 130a. Therefore, with the above design, better separation effect of the first component 210 and the second component 220 can be achieved.

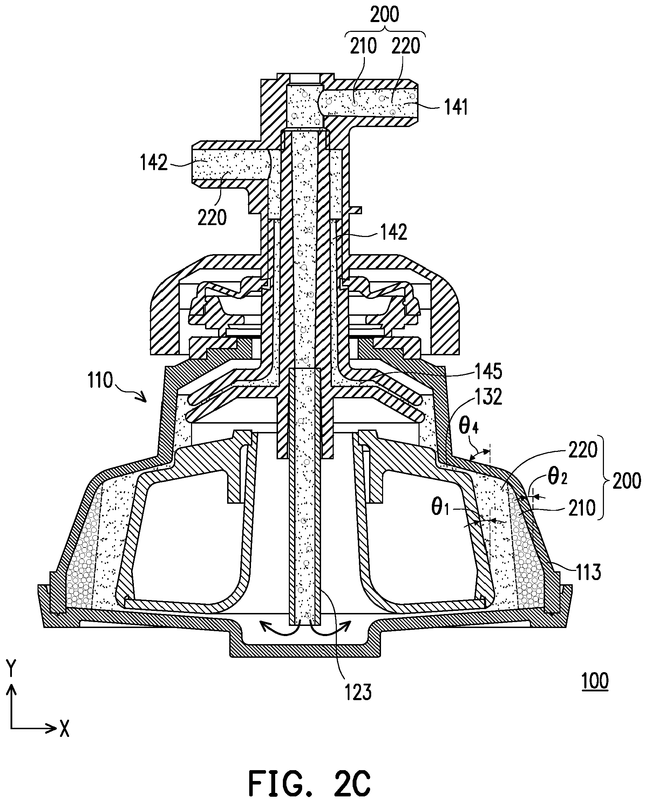

Next, referring to FIG. 2C, during centrifugation of the centrifuge bowl 100, the input of the sample 200 continues, so that the separated second component 220 can enter the second flow channel 132 and pass through the third flow channel 145 and the output tube 142 to be outputted and collected. Since the first angle θ₁ may be greater than 0 degrees and less than 90 degrees, the separated second component 220 can mostly enter the second flow channel 132. Since the fourth angle θ₄ may be greater than the second angle θ₂ and less than 90 degrees, the second flow channel 132 can be formed at the middle shell part 112, and the resistance to the second component 220 entering the second flow channel 132 is reduced.

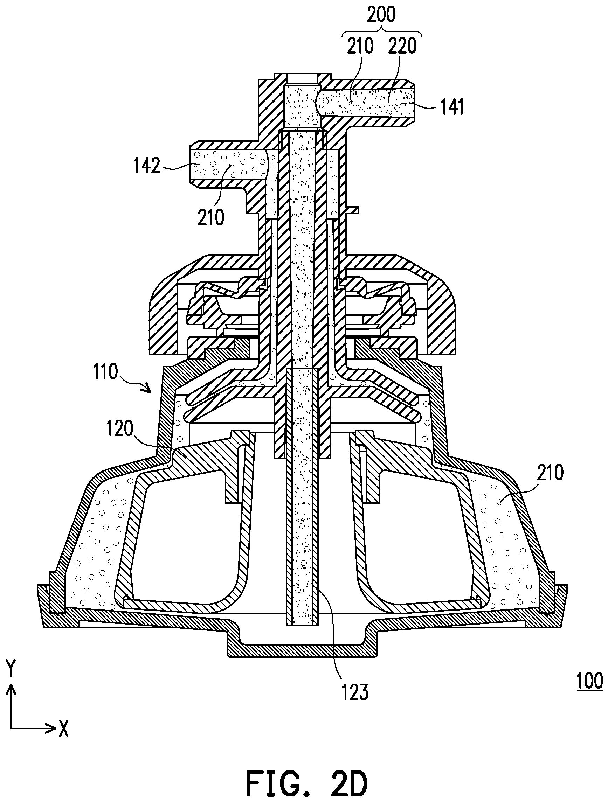

Next, referring to FIG. 2D, when collection of the second component 220 is completed or the first component 210 is detected to flow to the output tube 142, the input of the sample 200 may be stopped and the centrifugation may be stopped.

Other embodiments will be described below. It is noted herein that the reference numerals and part of the content of the foregoing embodiment will apply to the following embodiments. The same reference numerals are used to represent the same or similar elements, and the descriptions of the same technical contents will be omitted. Reference may be made to the foregoing embodiment for descriptions of the omitted parts, which shall not be repeated in the following embodiments.

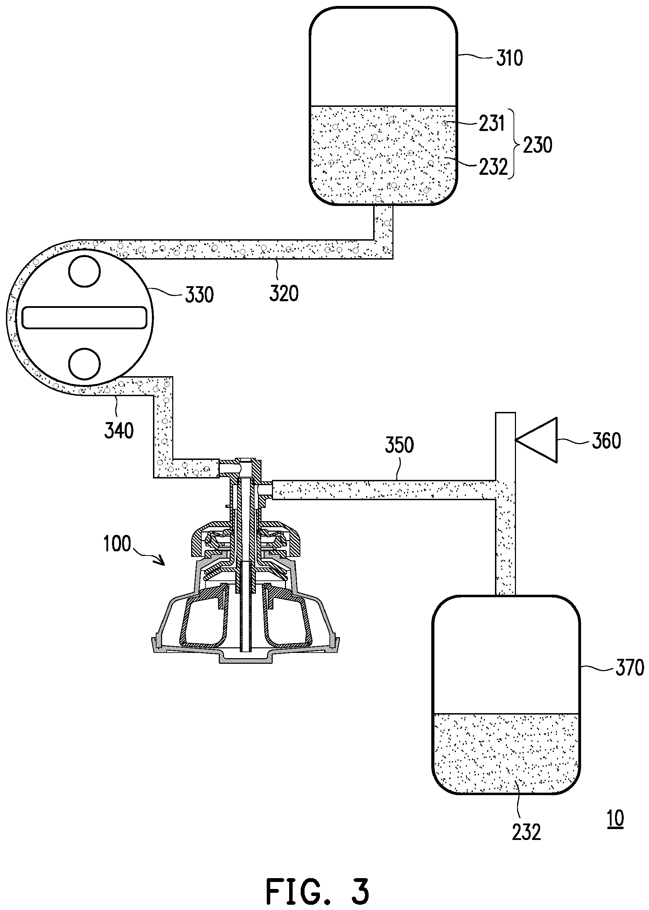

FIG. 3 is a schematic cross-sectional view of a blood centrifuge system according to an embodiment of the disclosure. Referring to FIG. 3, a blood centrifuge system 10 of this embodiment includes a first container 310, a first pipeline 320, a pump 330, a second pipeline 340, a centrifuge bowl 100, a third pipeline 350, a vent 360, and a second container 370. The first pipeline 320 connects the first container 310 and the inlet end of the pump 330. The second pipeline 340 connects the outlet end of the pump 330 and the input tube of the centrifuge bowl 100. The third pipeline 350 connects the centrifuge bowl 100 and the second container 370. The first container 310 may be configured to hold 60 to 100 milliliters (ml) of blood 230, and the blood 230 may include red blood cells 231 and plasma 232 containing platelets. The second container 370 may be configured to collect 10 to 50 ml of the plasma 232 containing platelets.

Specifically, for example, the blood centrifuge system 10 is configured to separate the plasma 232 containing platelets from the blood 230 by the following steps. First, the pump 330 is used to input the blood 230 in the first container 310 to the centrifuge bowl 100 via the first pipeline 320 and the second pipeline 340. Next, centrifugation of the centrifuge bowl 100 is performed at 2800 to 4500 RPM (revolution per minute) to separate the red blood cells 231 and the plasma 232 containing platelets in the blood 230. Finally, the separated plasma 232 containing platelets is outputted to the second container 370 via the third pipeline 350.

In this embodiment, since the centrifuge bowl 100 can automatically separate the red blood cells 231 and the plasma 232 containing platelets in the blood 230, the blood centrifuge system 10 of this embodiment can be fully automatically controlled to control the concentration and the collected amount of the plasma 232 containing platelets in the second container 370. In addition, since the centrifuge bowl 100 can automatically separate the red blood cells 231 and the plasma 232 containing platelets in the blood 230, the centrifuge bowl 100 can be designed as a fully hermetic centrifuge kit to thereby prevent the blood 230 from being contaminated by external sources. The collected plasma 232 containing platelets may be applied, for example, to treatment of ophthalmic diseases, but is not limited thereto.

Hereinafter, experimental examples will be described to illustrate the technical means adopted by the disclosure to achieve the objective. In the following examples, the sample is blood, but the disclosure is not limited thereto. In other words, those with ordinary skill in the art may easily replace the sample with other liquid samples based on the content disclosed in this embodiment.

### Experimental examples

The centrifuge bowl was used to automatically separate plasma containing platelets in blood. As shown in Table 1 below, plasma containing platelets may be separated from blood samples of different volumes and different hematocrits (HCT) at different centrifugal rotational speeds. The amount of collected plasma (containing platelets) and the multiple of the concentration of platelets in the plasma (containing platelets) are also shown in Table 1. The multiple refers to a ratio of the concentration of platelets in the collected plasma (containing platelets) to the concentration of platelets in the blood.

**Table 1**

| | Centrifugal rotational speed (RPM) | Blood volume (ml) | Hematocrit (%) | Collected amount (ml) | Multiple |
|---|---|---|---|---|---|
| Experimental example 1 | 3000±5% | 100 | 24 | 50 | 2.0±10% |
| Experimental example 2 | 3000±5% | 60 | 40 | 10 | 2.0±10% |
| Experimental example 3 | 4000±5% | 100 | 29 | 50 | 1.2±10% |
| Experimental example 4 | 4000±5% | 60 | 48 | 10 | 1.2±10% |

According to the results in Table 1, compared to the centrifugal rotational speed of 4000±5% RPM, when the centrifugal rotational speed was 3000±5% RPM, the multiple of the concentration of platelets in the collected plasma (containing platelets) was higher.

In summary of the above, in the centrifuge bowl and the blood centrifuge system according to an embodiment of the disclosure, due to the difference in the magnitude of the initial force of the first component and the second component in the sample, when the shell and the core rotate on the rotation axis, the sample can be automatically separated into the first component and the second component in the separation cavity according to the magnitude of the inertial force. Accordingly, the centrifuge bowl and the blood centrifuge system according to an embodiment of the disclosure can achieve the effect of automatically separating blood. In addition, since the second angle may be greater than the first angle, the second flow velocity of the first component flowing toward the second flow channel can be further reduced, so that the first component can more easily concentrate in the recessed region. Accordingly, better separation effect of the first component and the second component can be achieved.

### REFERENCE SIGNS LIST

- 10:: Blood centrifuge system
- 100:: Centrifuge bowl
- 110:: Shell
- 111:: Upper shell part
- 112:: Middle shell part
- 112a:: Outer surface
- 113:: Lower shell part
- 113a:: Outer surface
- 114:: Bottom shell part
- 114a:: Inner surface
- 115:: Opening
- 116:: Internal space
- 120:: Core
- 121:: Outer side-surface
- 122:: Outer bottom-surface
- 123:: Central sleeve
- 130:: Separation cavity
- 130a:: Recessed region
- 131:: First flow channel
- 132:: Second flow channel
- 140:: Stator head
- 141:: Input tube
- 142:: Output tube
- 143:: Sealing gasket
- 144:: Shell cover
- 145:: Third flow channel
- 200:: Sample
- 210:: First component
- 220:: Second component
- 310:: First container
- 320:: First pipeline
- 330:: Pump
- 340:: Second pipeline
- 350:: Third pipeline
- 360:: Vent
- 370:: Second container
- R:: Rotation axis
- X:: Second direction
- Y:: First direction
- θ₁:: First angle
- θ₂:: Second angle
- θ₃:: Third angle
- θ₄:: Fourth angle

## Claims

1. A centrifuge bowl (100) configured to separate a first component (210) and a second component (220) in a sample (200), the centrifuge bowl (100) comprising:
a shell (110) comprising an upper shell part (111), a middle shell part (112), a lower shell part (113), and a bottom shell part (114), wherein the middle shell part (112) connects the upper shell part (111) and the lower shell part (113), and the lower shell part (113) connects the middle shell part (112) and the bottom shell part (114);
a core (120) arranged in the shell (110);
a separation cavity (130) arranged between the lower shell part (113) and the core (120); and
a stator head (140) arranged on the shell (110) and comprising an input tube (141) and an output tube (142),
wherein the sample (200) enters the separation cavity (130) via the input tube (141), and
when the shell (110) and the core (120) rotate on a rotation axis (R), the sample (200) in the separation cavity (130) is separated into the first component (210) and the second component (220) according to a magnitude of an inertial force.

2. The centrifuge bowl (100) according to claim 1, wherein the separation cavity (130) is defined as being surrounded by the core (120), the middle shell part (112), the lower shell part (113), and the bottom shell part (114).

3. The centrifuge bowl (100) according to claim 1, wherein when a first inertial force of the first component (210) is greater than a second inertial force of the second component (220), the separated first component (210) is away from the core (120), and the separated second component (220) is adjacent to the core (120).

4. The centrifuge bowl (100) according to claim 1, wherein a first angle (θ₁) is present between an outer side-surface (121) of the core (120) and a first direction (Y) parallel to the rotation axis (R), a second angle (θ₂) is present between an outer surface (113a) of the lower shell part (113) and the first direction (Y), and the second angle (θ₂) is greater than the first angle (θ₁).

5. The centrifuge bowl (100) according to claim 4, wherein the first angle (θ₁) is less than 90 degrees, and the second angle (θ₂) is less than 90 degrees.

6. The centrifuge bowl (100) according to claim 4, wherein a third angle (θ₃) is present between an inner surface (114a) of the bottom shell part (114) and the first direction (Y), and the third angle (θ₃) is greater than 90 degrees.

7. The centrifuge bowl (100) according to claim 4, wherein a fourth angle (θ₄) is present between an outer surface (112a) of the middle shell part (112) and the first direction (Y), the fourth angle (θ₄) is less than 90 degrees, and the fourth angle (θ₄) is greater than the second angle (θ₂).

8. The centrifuge bowl (100) according to claim 1, wherein the separation cavity (130) has a recessed region (130a), and the recessed region (130a) is defined by a contour formed upon connection of the lower shell part (113) and the bottom shell part (114).

9. The centrifuge bowl (100) according to claim 1, wherein the sample (200) is blood, the first component (210) comprises red blood cells, and the second component (220) comprises platelets and plasma.

10. A blood centrifuge system (10) comprising the centrifuge bowl (100) according to claim 1.
